# EUROPÄISCHE PATENTANMELDUNG

(11) **EP 3 418 716 A1**
(43) Veröffentlichungstag der Anmeldung: **26.12.2018**
(21) Anmeldenummer: 18178969.4
(22) Anmeldetag: 21.06.2018
(51) Int. Cl.: G01N 11/02, G01N 11/10, G01N 33/44

(54) **VERFAHREN ZUR BESTIMMUNG DER TOPFZEIT**

(30) Priorität: 22.06.2017 AT 505142017
(71) Anmelder: IFN-Holding AG, 4050 Traun (AT)
(72) Erfinder: HACKL, Herbert, 4132 Lembach (AT); MAYRHOFER, Manuel, 4142 Hofkirchen (AT); PEER, Martin, 4141 Pfarrkirchen (AT)
(74) Vertreter: Burger, Hannes

(57) **Zusammenfassung**

Die Erfindung betrifft ein Verfahren zur Bestimmung des Endes der Topfzeit eines härtenden Harzes oder eines härtenden Gemisches aus einer Komponente A und einer Komponente B umfassend die Schritte:
- Gegebenenfalls vermischen der Komponente A mit der Komponente B zur Herstellung des Gemisches,
- Entnahme einer vorbestimmbaren Prüfmenge aus dem Harz oder dem Gemisch,
- Überführen der Prüfmenge in eine Messvorrichtung (1) zur Bestimmung der Topfzeit des Harzes oder des Gemisches,
- Eintauchen eines Messelementes (5) in vorbestimmbaren Zeitabständen in die Prüfmenge,
- anschließend daran Herausziehen des Messelementes (5) aus der Prüfmenge mit vorbestimmbarer Geschwindigkeit.

Beim Herausziehen des Messelementes (5) wird ein Faden (15) aus dem Harz oder dem Gemisch gezogen, der mit einem Sensorelement (8) erfasst wird, wobei der Vorgang des Eintauchens und Herausziehen des Messelementes (5) solange wiederholt wird, bis der Faden (15) beim Herausziehen aus dem Harz oder dem Gemisch zur Gänze und mit einer vordefinierbaren verbleibenden Länge am Messelement (5) abreißt.

## Beschreibung

Die Erfindung betrifft ein Verfahren zur Bestimmung (des Endes) der Topfzeit eines härtenden Harzes oder eines härtenden Gemisches aus einer Komponente A und einer Komponente B umfassend die Schritte: Gegebenenfalls vermischen der Komponente A mit der Komponente B zur Herstellung des Gemisches, Entnahme einer vorbestimmbaren Prüfmenge aus dem Harz oder dem Gemisch, Überführen der Prüfmenge in eine Messvorrichtung zur Bestimmung der Topfzeit des Harzes oder des Gemisches, Eintauchen eines Messelementes in vorbestimmbaren Zeitabständen in die Prüfmenge, anschließend daran Herausziehen des Messelementes aus der Prüfmenge mit vorbestimmbarer Geschwindigkeit.

Weiter betrifft die Erfindung eine Messvorrichtung zur Durchführung des Verfahrens zur Bestimmung (des Endes) der Topfzeit eines härtenden Harzes oder eines härtenden Gemisches aus einer Komponente A und einer Komponente B umfassend: eine Halterung für ein Aufnahmegefäß, in das eine Prüfmenge des Harzes oder des Gemisches einfüllbar ist, ein Halteelement für ein Messelement, eine Halteeinrichtung, auf der das Halteelement beweglich angeordnet ist, ein Antriebselement mit dem das Halteelement verstellbar ist und ein Sensorelement.

Zudem betrifft die Erfindung ein Verfahren zur Steuerung einer Vorrichtung zur Verarbeitung eines härtenden Gemisches aus einer Komponente A und einer Komponente B.

Unter Topfzeit versteht man die Verarbeitbarkeitsdauer von reaktiven Materialien, wie Lacken, Klebstoffe, Reaktivharzgemische. Sie wird auch als Offenzeit oder Gebrauchsdauer bezeichnet. Die Topfzeit ist also die Zeit zwischen dem Vermischen einer zweikomponentigen Substanz und dem Ende ihrer Verarbeitbarkeit, also die Zeitspanne, in der sich die Substanz noch aus dem Topf nehmen und verarbeiten lässt. Meist zeigt sich das Ende der Topfzeit durch deutlichen Viskositätsanstieg, der eine weitere Verarbeitung verhindert.

Die Topfzeit steht meist in direktem Zusammenhang mit der Gelzeit. Die Gelzeit schließt direkt an die Topfzeit im engeren Sinne an und bezeichnet ebenfalls das Verarbeitungsfenster der zweikomponentigen, aushärtenden Masse. Aus diesem Grund werden die Begriffe "Topfzeit" und "Gelzeit" häufig synonym verwendet.

Dem schließt sich die vorliegende Beschreibung an. Im Folgenden wird daher der Begriff "Topfzeit" und "Gelzeit" häufig synonym für die Zeitspanne der Verarbeitbarkeit des Gemisches aus den Komponenten A und B verwendet.

In der Regel kann die Topfzeit eingestellt werden durch die Compoundierung der Mischung. Als Maß für die Topfzeit wird dabei die Viskosität der Mischung herangezogen. Anhand des Anstieges der Viskosität aufgrund der voranschreitenden Härtung des Gemisches wird die Topfzeit bestimmt.

So ist es beispielsweise bei Lacken üblich, zur Bestimmung der Topfzeit ein spezielles Gefäß mit einem festgelegten Volumen (Norm-Auslaufbecher) mit der Mischung zu füllen. Das Gefäß weist im Boden ein Loch auf, aus dem die Mischung herausläuft. Gemessen wird die Auslaufzeit. Die Topfzeit ist bestimmt durch den Anstieg der Auslaufzeit um einen bestimmten Faktor, in der Regel auf das Doppelte des Anfangswerts. Andere Messverfahren, die der Lackindustrie Anwendung finden, sind beispielsweise in der DE 102 36 122 A1 genannt.

Zur Messung der Gelierzeit von Reaktivharzmischungen sind auch Geräte unter der Bezeichnung GELNORM® Gel Timer bekannt. Dabei wird ein Messstempel in ein Reagenzglas eingetaucht, das die Reaktivharzmischung enthält. Der Stempel führt anschließend Hubbewegungen durch. Steigt die Viskosität über einen bestimmten Wert an, wird der Stempel in dem Reagenzglas gehalten. Durch die folgende Hubbewegung wird dann das Reagenzglas angehoben. Dies markiert das Ende der Gelierzeit. Zur automatischen Messung kann ein optischer Sensor vorgesehen sein, sodass das Gerät nach dem Ende der Gelierzeit automatisch abschaltet. Das Reagenzglas und der Messstempel werden nach der Messung entsorgt, sodass keine Reinigung notwendig ist.

Der vorliegenden Erfindung liegt die Aufgabe zu Grunde, die Messung der Topfzeit unter industriellen Bedingungen möglichst einfach durchführen zu können. Insbesondere ist es eine weitere Aufgabe der Erfindung, die Messergebnisse aus der Bestimmung besser in die industrielle Fertigung einbinden zu können.

Die Aufgabe der Erfindung wird mit dem eingangs genannten Verfahren dadurch gelöst, dass beim Herausziehen des Messelementes ein Faden aus dem Harz oder dem Gemisch gezogen wird, der mit einem Sensorelement erfasst wird, wobei der Vorgang des Eintauchens und Herausziehen des Messelementes solange wiederholt wird, bis der Faden beim Herausziehen aus dem Harz oder dem Gemisch zur Gänze und mit einer vordefinierbaren verbleibenden Länge am Messelement abreißt.

Weiter wird die Aufgabe der Erfindung mit der eingangs genannten Messvorrichtung zur Durchführung des genannten Verfahrens gelöst, bei der das Halteelement um eine Länge von mindestens 10 cm verstellbar ist.

Die Aufgabe wird zudem mit dem voranstehend genannten Verfahren zur Steuerung einer Vorrichtung zur Verarbeitung eines härtenden Gemisches aus einer Komponente A und einer Komponente B gelöst, wonach mit der Messvorrichtung und nach dem Verfahren die Topfzeit des Gemisches bestimmt wird und in Anhängigkeit der bestimmten Topfzeit des Gemisches die Zufuhr der Menge der Komponente A und der Menge der Komponente B zur Vorrichtung zur Verarbeitung des härtenden Gemisches aus der Komponente A und der Komponente B gleichbelassen oder verändert wird.

Von Vorteil ist dabei, dass Verfahren einfach durchführbar ist, und damit auch von Personen durchgeführt werden kann, die lediglich geringe Erfahrungen im Bereich von Routinemessungen in der industriellen Fertigung haben. Im Wesentlichen muss nur das Aufnahmegefäß für das Gemisch aus den beiden Komponenten richtig in der Messvorrichtung platziert werden. Die eigentliche Messung kann dann automatisiert erfolgen, wodurch Fehler aufgrund manueller Bewertungen reduziert bzw. ausgeschlossen werden können. Der menschliche Einfluss auf das Messergebnis kann damit minimiert werden. Mit der Messmethode ist es nicht notwendig, dass das Messelement tief in das Aufnahmegefäß, d.h. in das darin enthaltene Gemisch aus den beiden Komponenten A und B, eintaucht. Dies wiederum ist von Vorteil in Hinblick auf den ungestörten Strukturaufbau des regierenden, d.h. härtenden, Gemisches, wodurch Fehler, die z.B. aufgrund einer zusätzlichen Durchmischung des Gemisches im Aufnahmegefäß mit dem Messelement entstehen könnten, besser vermieden werden. Die Messung kommt daher der Realität in der Verarbeitung der des Gemisches näher, wodurch realistischere Messwerte erzielt werden können. Darüber hinaus bietet die Messmethode auch eine bessere Möglichkeit der visuellen Kontrolle des Fortschritts der Härtung des Gemisches, da anhand der Geometrie (bzw. der Geometrieänderung über den Zeitverlauf) des gezogenen Fadens abgeschätzt werden kann, wie lange die restliche Topfzeit noch ist. Dies ist beispielsweise mit dem voranstehend genannten GELNORM® Gel Timer nicht bzw. nur schwer möglich.

Mit dem Verfahren zur Steuerung einer Vorrichtung zur Verarbeitung eines härtenden Gemisches kann basierend auf den gemessenen bzw. bestimmten Messwert automatisch auf die Dosierung der Komponenten A und B Einfluss genommen werden, sodass Fehler in der Verarbeitung, die durch zu geringe Topfzeit entstehen können, reduziert werden können.

Nach einer bevorzugten Ausführungsvariante des Verfahrens kann vorgesehen sein, dass als Sensorelement ein optisches Sensorelement verwendet wird, wozu nach einer Ausführungsvariante der Messvorrichtung das Sensorelement ein optisches Sensorelement ist. Es ist damit eine berührungslose Erfassung des Vorhandenseins des Fadens bzw. von dessen Geometrie im Messfeld des Sensorelementes möglich, wodurch mit dem Sensorelement kein Einfluss auf den Reaktionsablauf im Gemisch genommen wird und somit die Messgenauigkeit verbessert werden kann. Zudem kann damit auch eine Verschmutzung des Sensorelementes vermieden werden, sodass die Messvorrichtung nach einer durchgeführten Messung relativ rasch für eine neue Messung vorbereitet werden kann.

Es kann weiter vorgesehen sein, dass mit dem Sensorelement die Geometrie des Fadens im Bereich des Sensorelementes vermessen wird. Durch die Geometrievermessung kann besser auf die verbleibende Topfzeit rückgeschlossen werden, da der Querschnitt bzw. die Länge des gezogenen Fadens mit zunehmender Härtung des Gemisches kleiner wird.

Bevorzugt wird nicht nur das Ende der Topfzeit nach dem Verfahren bestimmt, sondern wird die Topfzeit aus der Zeitspanne zwischen der Entnahme des Harzes oder dem Mischen der Komponente A mit der Komponente B und dem detektierten Ende der Topfzeit errechnet. Bevorzugt kann dabei nach einer Ausführungsvariante des Verfahrens vorgesehen sein, dass die Zeitspanne, die zwischen der Entnahme des Harzes oder der Mischung der Komponente A mit der Komponente B und dem Beginn der ersten Messung dieses Harzes oder Gemisches vergangen ist, in einem elektronischen Speicher hinterlegt und in die Berechnung der Topfzeit einbezogen wird. Es kann damit auf eine definierte Zeitvorgabe, innerhalb der das Harz oder das Gemisch nach dem Vermischen der Komponenten in die Messvorrichtung gegeben werden muss, verzichtet werden. Damit kann die Messung für weniger routinierte Mitarbeiter einfacher gestaltet werden, wodurch wiederum die industrielle Einsetzbarkeit der Messvorrichtung und des Verfahrens zur Messung der Topfzeit vereinfacht werden kann.

Bevorzugt wird mit zumindest einem Temperatursensor die Umgebungstemperatur der Messvorrichtung und/oder die Temperatur beim Mischen der Komponente A mit der Komponente B erfasst und/oder wird nach einer weiteren Ausführungsvariante des Verfahrens mit zumindest einem Feuchtigkeitssensor die Luftfeuchtigkeit der Umgebung der Messvorrichtung und/oder der Luftfeuchtigkeit beim Mischen der Komponente A mit der Komponente B erfasst. Zu diesem Zweck weist die Messvorrichtung nach einer Ausführungsvariante zusätzlich zumindest einen Feuchtigkeitssensor und/oder zumindest einen Temperatursensor auf. Durch die Mitberücksichtigung von zumindest einem dieser Parameter kann die Qualität des Messergebnisses verbessert werden. In weiterer Folge kann damit das Messergebnis besser auf die vorherrschenden Bedingungen in der Fertigungsstraße, in der das Gemisch aus den Komponenten A und B verarbeitet wird, umgelegt werden.

Nach einer Ausführungsvariante der Messvorrichtung kann vorgesehen sein, dass das optische Sensorelement eine Kamera mit einem CCD-Sensor ist. Es ist damit möglich, nicht nur Messwerte sondern Bilder des Messvorgangs an einem Monitor übertragen werden, der in einer größeren Entfernung von der Messvorrichtung steht, beispielsweise bei der Fertigungsstraße, in der das Gemisch aus den Komponenten A und B verarbeitet wird. Damit wird einem dort arbeitenden Mitarbeiter auf einfache Weise ermöglicht, rasch den Status der Messung der Topfzeit zu erfassen und gegebenenfalls Verarbeitungsparameter für das Gemisch zu ändern.

Bevorzugt weist die Messvorrichtung zumindest einen elektronischen Speicher auf, um beispielsweise die Messung abzuspeichern. Nach einer bevorzugten Ausführungsvariante der Messvorrichtung dazu kann jedoch vorgesehen sein, dass der elektronische Speicher mit einem Eingabeelement verbunden ist, über das die Zeitspanne eingegeben werden kann, die zwischen dem Mischen der Komponente A mit der Komponente B und dem Beginn der ersten Messung vergangen ist. Es sei dazu auf die voranstehend genannte Ausführungsvariante des Verfahrens verwiesen.

Weiter kann die Messvorrichtung eine Mischvorrichtung für die Vermischung der Komponente A mit der Komponente B aufweisen. Es kann damit die Mischung der Komponenten A und B erst in der Messvorrichtung selbst erfolgen, sodass die während der Härtung des Gemisches ablaufende chemische Reaktion im Wesentlichen erst unmittelbar vor dem Start der Messung der Topfzeit beginnt.

Nach einer anderen Ausführungsvariante der Messvorrichtung kann vorgesehen sein, dass diese eine Zufuhrleitung für die Komponente A und eine Zufuhrleitung für die Komponente B zur Zufuhr der beiden Komponenten A und B in das Aufnahmegefäß aufweist. Mit dieser Ausführungsvariante kann der Automatisierungsgrad der Messung der Topfzeit des Gemisches weiter erhöht werden.

Zum besseren Verständnis der Erfindung wird diese anhand der nachfolgenden Figuren näher erläutert.

Es zeigen jeweils in vereinfachter, schematischer Darstellung:
- Fig. 1: eine Ausführungsvariante einer Messvorrichtung im Querschnitt;
- Fig. 2: einen Ausschnitt aus einer Messvorrichtung in Seitenansicht zur Verdeutlichung des Messprinzips;
- Fig. 3: eine Explosionsdarstellung einer Messvorrichtung in Schrägansicht;
- Fig. 4: Darstellungen von Messelementen mit gezogenem Faden der gemessenen Probe im Verlauf einer Messung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

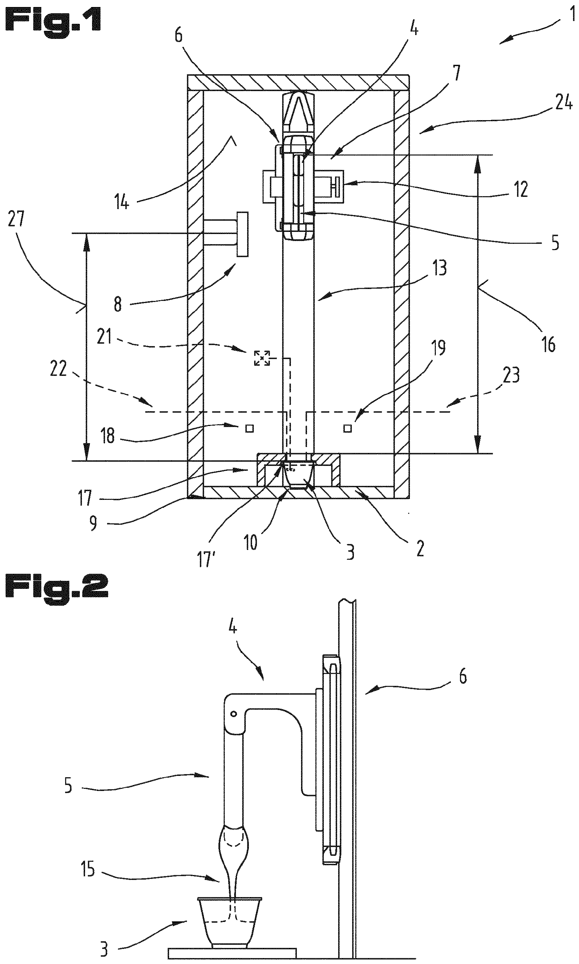

In Fig. 1 ist eine Ausführungsvariante einer Messvorrichtungl zur Messung des Endes der Topfzeit eines härtenden Gemisches aus einer Komponente A und einer Komponente B im Querschnitt und in Frontansicht dargestellt.

Das härtende Gemisch kann beispielsweise ein Lack oder ein, insbesondere lösungsmittelfreier, Klebstoff oder ein, insbesondere lösungsmittelfreies, Reaktivharzgemisch sein. Beispielsweise kann das Gemisch ein ungesättigte Polyesterharz, ein Epoxidharz, ein Polyurethanharz, ein (Meth)Acrylatharz oder ein Silikonharz sein.

Die Messvorrichtung 1 umfasst eine Halterung 2 für ein Aufnahmegefäß 3, ein Halteelement 4 für ein Messelement 5, eine Halteeinrichtung 6, auf der das Halteelement 4 beweglich, insbesondere linear beweglich, angeordnet ist, ein Antriebselement 7 mit dem das Halteelement 4 verstellbar, insbesondere linear verstellbar, ist, und ein Sensorelement 8.

Anstelle der Linearbeweglichkeit des Halteelementes 4 kann auch eine Dreh- bzw. Schwenkbeweglichkeit des Halteelementes 4 vorgesehen werden bzw. sein.

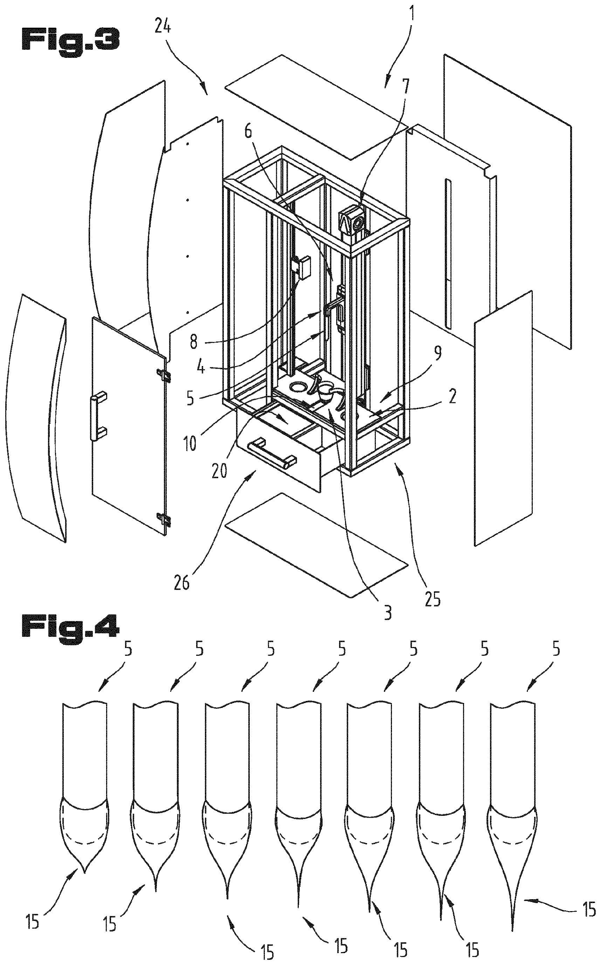

Die Halterung 2 für das Aufnahmegefäß 3 kann im einfachsten Fall durch einen Boden 9 gebildet sein, auf den das Aufnahmegefäß 3 gestellt wird. Zur genaueren Positionierung des Aufnahmegefäßes 3 kann der Boden 9 eine Ausnehmung 10 aufweisen. Diese Ausnehmung 10 kann der Geometrie des Bodens des Aufnahmegefäßes 3 angepasst sein, d.h. dass die Ausnehmung 10 eine Aufstandsfläche für das Aufnahmegefäß aufweist, die genau so groß ist oder um maximal 10 %, insbesondere maximal 5 % größer, ist, als die Aufstandsfläche des Bodens des Aufnahmegefäßes 3. In diesem Fall ist die Ausnehmung 10 unterhalb des Messelementes 5 und in der Flucht des Messelementes 5 im Boden 9 ausgebildet. Vorzugsweise ist die Ausnehmung 10 aber nutförmig ausgebildet, wie dies aus Fig. 3 ersichtlich ist, die eine weitere Ausführungsvariante der Messeinrichtung 1 in Explosionsdarstellung zeigt. Die nutförmige Ausnehmung 10 erstreckt sich dabei vorzugsweise von der Vorderseite der Messvorrichtung 1, d.h. von einer vorderen Stirnfläche 11 des Bodens 9, bis in den Bereich unterhalb und in der Flucht des Messelementes 5. Mit der nutförmigen Ausnehmung 10 wird dabei nicht nur die genaue Positionierung des Aufnahmegefäßes 3 unterhalb des Messelementes 5 ermöglicht, sondern kann damit das Aufnahmegefäß 3 auch einfacher in die Messeinrichtung 1 eingeführt werden, indem es lediglich in der nutförmigen Ausnehmung 10 nach hinten in die Messposition unterhalb und in der Flucht des Messelementes 5 geschoben wird.

Das Aufnahmegefäß 3 ist bevorzugt als Becher ausgeführt. Weiter kann das Aufnahmegefäß 3 einen runden oder quadratischen oder rechteckförmigen, etc., Querschnitt aufweisen, wobei die runde Querschnittsform bevorzugt ist. Zudem kann das Aufnahmegefäß 3 mit einem kegelstumpfförmigen Mantel ausgebildet sein, wie dies in Fig. 1 dargestellt ist, bzw. generell sich in Richtung auf den Boden 9 sich verjüngend ausgebildet sein, oder zylinderförmig oder würfelförmig bzw. quaderförmig, etc. Das Aufnahmegefäß 3 ist oben in Richtung auf das Messelement 5 offen ausgeführt, wobei auch eine teilweise Abdeckung des Aufnahmegefäßes 3 möglich ist. In letzterem Fall ist das Aufnahmegefäß 3 zumindest in einem Bereich offen ausgebildet, in dem das Messelement 5 für die Messung der Topfzeit in das Aufnahmegefäß 3 eintaucht.

Das Haltelement 4 für das Messelement 5 ist bevorzugt in Form von Klemmbacken ausgeführt, sodass das Messelement 5 einfach in dem Haltelement 4 durch Klemmung angeordnet werden kann. Zum Öffnen und Schließen der Klemmbacken kann Betätigungselement 12 vorgesehen sein, das mit einem der beiden Klemmbacken verbunden ist. Das Betätigungselement 12 kann beispielsweise eine (Rändel-)Schraube oder ein Kniehebelspanner oder ein anderes, geeignetes Spannelement sein.

Es ist weiter möglich, dass die Klemmbacken zumindest im Bereich der Anlage des Messelementes 5 eine Oberflächenaufrauhung und/oder eine Beschichtung bzw. eine Schicht zur Erhöhung der Reibung zwischen Klemmbacken und Messelement 5 aufweist.

Das Halteelement 4 für das Messelement 4 kann aber auch anders ausgeführt sein, beispielsweise kann das Messelement 4 mit dem Haltelement 4 verschraubt sein, wobei generell eine Spannvorrichtung bevorzugt ist, da damit ein schneller Wechsel des Halteelements 4 ermöglicht wird.

Das Halteelement 4 und damit das davon gehaltene Messelement 5 sind (linear) beweglich in der Messvorrichtung 1 angeordnet. Dazu ist das Haltelement 4 auf der Halteinrichtung 6 angeordnet. Die Halteeinrichtung 6 kann beispielsweise ein schienenförmiges Profilelement 13 eines Antriebes, insbesondere eines Linearantriebes, sein. Dabei kann das Haltelement 4 das Profileelement 13 außen umgreifen oder in einer Nut des Profilelementes 13 geführt sein. Ebenso ist es möglich, dass das Profilelement 12 einen Führungssteg aufweist, auf dem das Haltelement angeordnet ist. Derartige Linearantriebe sind prinzipiell bekannt, sodass zu weiteren Einzelheiten dazu auf den einschlägigen Stand der Technik zu Linearantriebe verwiesen sei.

Gegebenenfalls kann das Halteelement 4 zusätzlich zur Linearbeweglichkeit drehbeweglich auf der Halteeinrichtung 6 angeordnet sein, um das Messelement 5 in Drehbewegung zu versetzen. Dies kann beispielsweise vorgesehen sein, wenn das Messelement 5 auch zur Vermischung der Komponenten A und B im Aufnahmegefäß 3 verwendet wird. Die Drehbewegung kann aber auch dazu vorgesehen werden, um damit eine bessere Haftung der Probe auf dem Messelement 5 zu erreichen. Das Halteelement 4 kann dazu einen entsprechenden Drehantrieb aufweisen oder mit diesem verbunden sein.

Für eine bessere Haftung der Probe auf dem Messelement 5 kann auch ein Vibrationsantrieb, der die Probe in Vibration versetzt, verwendet werden.

Die Halteeinrichtung 6 kann an einer Rückwand 14 der Messeinrichtung 1 moniert sein. Es ist aber auch möglich, dass die Halteeinrichtung 6 freistehend in der Messeinrichtung 1 angeordnet ist. Jedenfalls ist die Halteeinrichtung 6 in der Messeinrichtung 1 so platziert, dass das Messelement 5 beim Abwärtsbewegen des Halteelements 4 in das Aufnahmegefäß 3 eintaucht.

Für die (lineare) Verstellbarkeit des Halteelementes 4 ist das Antriebselement 7 vorgesehen, das beispielsweise ein Elektromotor ist. Das Antriebselement 7 kann Teil des voranstehend genannten Antriebs, insbesondere des Linearantriebs, sein.

Die Übertragung der Bewegung vom Antriebselement 7 auf das Haltelement 4 kann beispielsweise mittels eines Zahnriemens oder mit einer Spindel oder magnetisch oder pneumatisch erfolgen. Je nach Antriebsart sind das Haltelement 3 und das Antriebselement 7 entsprechend ausgebildet. Die Bewegung, insbesondere die Linearbewegung, des Halteelements 4 sollte bevorzugt mit einer definierten bzw. vordefinierbaren, gegebenenfalls gleichförmigen, Geschwindigkeit erfolgen. Es ist dabei möglich, dass die Höhe der Geschwindigkeit an der Messeinrichtung 1 einstellbar ist, sodass unterschiedliche Geschwindigkeiten für verschiedene Gemische aus den Komponenten A und B realisiert werden können. Es kann aber auch ein Geschwindigkeitsprofil für eine Messung bzw. eine Messserie verwendet werden.

Obwohl das Antriebselement 7 in Fig. 1 auf der Höhe des Halteelements 4 dargestellt ist, kann das Antriebselement 7 in der Messvorrichtung 1 auch an einem anderen Ort platziert sein und mit entsprechenden Leitungen mit dem Haltelement 4 oder der Halteeinrichtung 6 zur Übertragung der Bewegung auf das Halteelement 4 verbunden sein.

Das Messelement 5 ist bevorzugt als Messspatel ausgeführt. Es kann aus Holz oder aus Kunststoff oder aus Metall bestehen. Eine Messspatel aus Holz hat den Vorteil, dass eine Reinigung des Messelementes 5 nach erfolgter Messung nicht durchgeführt werden muss, sondern das Messelement 5 nach erfolgter Messung verworfen wird. Vorzugsweise wird jedoch eine Messspatel aus Kunststoff eingesetzt.

Das Messelement 5 kann auch eine andere als die flache Spatelgeometrie aufweisen, beispielsweise als Rundstab ausgeführt sein. Generell ist eine stabförmige Ausführung des Messelementes 5 bevorzugt.

Es sei erwähnt, dass auch das Aufnahmegefäß 3 bevorzugt nach erfolgter Messung nicht gereinigt sondern verworfen wird. Aus diesem Grund wird vorzugsweise ein Aufnahmegefäß 3 aus Kunststoff eingesetzt.

Die Messspatel kann beispielsweise 15 cm x 1,9 cm x 0,2 cm (L x B x H) groß sein.

Es sind aber auch anders ausgebildete Messelemente 5 einsetzbar, beispielsweise stabförmige mit runden oder viereckigen oder mehreckigem Querschnitt. Das Messelement 5 ist vorzugsweise so steif, dass es sich beim Eintauchen in das Gemisch aus den beiden Komponenten A und B nicht verbiegt.

Wie erwähnt, wird zur Bestimmung der Topfzeit das Messelement 5 in das Gemisch aus den beiden Komponenten A und B eingetaucht. Dazu ist nach dem Verfahren zur Bestimmung des Endes der Topfzeit des härtenden Gemisches aus der Komponente A und der Komponente B vorgesehen, dass in einem ersten Schritt die beiden Komponenten A und B miteinander zur Herstellung des Gemisches vermischt werden. Aus diesem Gemisch wird anschließend daran eine vorbestimmbare Prüfmenge des Gemisches entnommen. Die Prüfmenge kann beispielsweise zwischen 20 g und 100 g, z.B. 50 g, betragen. Diese Prüfmenge wird in das Aufnahmegefäß 3 eingefüllt und damit in die Messvorrichtung 1 überführt. Danach wird die Messung gestartet. Zur Messung wird das auf dem Halteelement 5 bereits vormontierte Messelement 5 mittels des Antriebselements 7 (linear) aus seiner oberen Ruhestellung nach unten in Richtung auf das Aufnahmegefäß 3 bewegt. Diese Bewegung, insbesondere Linearbewegung, erfolgt solange, bis das Messelement 5 in das Gemisch aus den beiden Komponenten A und B im Aufnahmegefäß 3 eintaucht. Die Eintauchtiefe kann zwischen 5 mm und 40 mm, z.B. 20 mm, betragen. Anschließend wird die Bewegungsrichtung des Antriebselementes 7 umgekehrt und das Messelement aus dem Gemisch aus den beiden Komponenten A und B wieder herausgezogen. Beim Herausziehen des Messelementes 5 wird ein Faden 15 aus dem Gemisch aus den beiden Komponenten A und B gezogen, wie dies in Fig. 2 dargestellt ist. Der Faden 15 entsteht aufgrund der Haftung des Gemisches aus den beiden Komponenten A und B auf dem Messelement 5 und bedingt durch die noch niedrigere Viskosität des noch nicht ausgehärteten Gemisches.

Es sei erwähnt, dass angegebene Werte zu Probemenge, Eintauchtiefe, etc., keinen einschränkenden sondern nur beispielhaften Charakter haben.

Der Vollständigkeit halber sei an dieser Stelle erwähnt, dass der Begriff "Faden 15" nicht im Sinne eines Fadens zu verstehen ist, aus dem Gewebe hergestellt werden können. Der Begriff definiert vielmehr den Umstand, dass ein Teil des Gemisches aufgrund der "Klebrigkeit" (beding durch den Wert der Viskosität) von dem Messelement beim Herausziehen aus dem Gemisch mitgenommen wird und dabei in Folge der Einschnürung teilweise relativ dünn ausgezogen wird (= Fadenziehen).

Wie ausgeführt, wird für die Messung das Messelements 5 mit dem Haltelement 4 auf der Halteeinrichtung 6 (linear) aus der oberen Ruheposition in die untere Messposition, bei der das Messelement 5 in das Gemisch eintaucht, bewegt und anschließend wieder aus dem Gemisch herausgezogen. Eine Länge 16, über die das Halteelement 4 (linear) verstellbar ist, beträgt dazu mindestens 10 cm. Beispielsweise kann diese Länge zwischen 10 cm und 50 cm, insbesondere zwischen 15 cm und 30 cm, betragen. Die während der Messung durchgeführte tatsächliche (lineare) Verstellung des Halteelementes 4 mit dem Messelement 5 ist abhängig von dem jeweils konkret zu messenden Gemisches.

Der Vorgang des Herausziehens und wieder Eintauchens des Messelementes 5 in bzw. aus dem Gemisch wird solange wiederholt, bis beim Herausziehen der Faden 15 so reißt, dass der gezogene Faden kurz unterhalb des Messelementes 5 reißt. Es sei dazu auch auf die nachfolgenden Ausführungen zur Fig. 4 verwiesen. Das Reißen ist bedingt durch die ansteigende Viskosität des Gemisches aus den Komponenten A und B aufgrund fortschreitender Härtung. Das Reißen des Fadens 15 markiert das Ende der Topfzeit, also jener Zeit, innerhalb der das Gemisch verarbeitbar ist.

Der Faden 15, d.h. das Vorhandensein des Fadens 15, und das Reißen des Fadens 15, also das nicht mehr Vorhandensein des Fadens 15, wird mit dem zumindest einen Sensorelement 8 erfasst. Prinzipiell kommt jedes dafür geeignete Sensorelement 8 in Frage, beispielsweise ein kapazitives Sensorelement 8, das die Änderung der Kapazität aufgrund des Vorhandseins des Fadens 15, der als Dielektrikum wirkt, misst. Bevorzugt ist das Sensorelement 8 aber ein optisches Sensorelement 8. Beispielsweise kann das Sensorelement 8 eine Lichtschranke sein, die erkennt, wenn der Faden 15 im Lichtstrahl ist. Wird beim Ziehen des Fadens 15 die Lichtschranke nicht mehr unterbrochen, kann die Elektronik der Messeinrichtung 1 daraus auf das Ende der Topfzeit schließen. Das Sensorelement 8 kann auch durch einen Laser gebildet sein.

Besonders bevorzugt wird als optisches Sensorelement 8 aber eine Kamera mit CCD-Sensor verwendet. Es kann damit nicht nur relativ einfach das Ende der Topfzeit erkannt werden, sondern ist es darüber hinaus mit diesem Sensorelement 8 relativ einfach möglich, die Geometrie des Fadens 15 im Messfeld des Sensorelementes 8 zu bestimmen. Damit kann beispielsweise durch die Abnahme der Länge des Fadens 15 auf das baldige Ende der Topfzeit geschlossen werden. Es kann damit also der Verlauf der Härtung des Gemisches aus den Komponenten A und B besser dokumentiert werden.

Generell kommen als Sensorelement 8 Sensoren in Einsatz wie sie an sich bekannt sind. Eine weitere Ausführung der Details dazu, beispielsweise hinsichtlich des Aufbaus des Sensorelementes 8, erübrigt sich somit. Beispielsweis wird als Sensorelement 8 ein Cognex In-Sight 2000-120 der Cognex Corporation eingesetzt.

Nach einer Ausführungsvariante der Messvorrichtung 1 kann vorgesehen sein, dass die Halterung 2 für das Aufnahmegefäß 3 nicht nur durch den Boden 9 gebildet ist, sondern dass zusätzlich dazu ein Stützelement 17 vorgesehen ist, das das Aufnahmegefäß 3 zusätzlich an seinem Mantel abstützt. Insbesondere wird das Aufnahmegefäß 3 an seinem oberen, offenen Ende, also im Bereich oberen Randes des Aufnahmegefäßes 3, zusätzlich abgestützt, wie dies aus Fig. 1 ersichtlich ist. Das Stützelement 17 kann domförmig ausgebildet sein, wobei eine vordere Seite offen ausgebildet sein kann, um damit das Aufnahmegefäß 3 einfacher in das Stützelement 17 einführen zu können. Es ist weiter möglich, dass das Stützelement 17 den oberen Rand des Aufnahmegefäßes 3 überdeckt, wozu das Stützelement 17 in diesem Bereich der Anlage an dem Aufnahmegefäß 3 eine Ausnehmung 17' (Absetzung) aufweisen kann.

Wenn ein Aufnahmegefäß 3 verwendet wird, das einen oberen nach außen gebogenen Rand aufweist, besteht weiter die Möglichkeit, dass das Stützelement 17 mit einer Nut ausgebildet ist, in die dieser Rand des Aufnahmegefäßes 17 eingeschoben werden kann.

Mit dem zusätzlichen Stützelement 17 kann eine stabilere Halterung des Aufnahmegefäßes 3 erreicht werden.

Nach einer anderen Ausführungsvariante der Messvorrichtung 1 kann vorgesehen sein, dass als Halterung 2 für das Aufnahmegefäß 3 ausschließlich das Stützelement 17 verwendet wird. Dieses kann dann auf einer Höhe innerhalb der Messeinrichtung 1 angeordnet sein, dass das Aufnahmegefäß 3 in das Stützelement 17 eingehängt werden kann und somit nicht mehr auf dem Boden 9 aufsteht, wie dies bei den voranstehend beschriebenen Ausführungsvarianten der Messvorrichtung 1 der Fall ist. In der Ausführung der aufgehängten bzw. eingehängten Halterung des Aufnahmegefäßes 3 kann das Stützelement wieder eine Nut aufweisen, in ein oberer, nach außen umgebogener Rand des Aufnahmegefäßes 3 eingeschoben wird. Es besteht aber auch die Möglichkeit, dass das Stützelement einen Durchbruch aufweist, in den das Aufnahmegefäß 3 von oben eingeführt werden kann. Damit das Aufnahmegefäß 3 nicht durchrutscht, kann dieses einen konisch sich verjüngende Körper aufweisen, beispielsweise kegelstumpfförmig ausgebildet sein. Ebenso kann ein oberer, nach außen umgebogener Rand des Aufnahmegefäßes 3 dazu verwendet werden.

Nach weiteren Ausführungsvarianten der Messvorrichtung 1 kann vorgesehen sein, dass diese zusätzlich zumindest einen Feuchtigkeitssensor 18 und/oder zumindest einen Temperatursensor 19 aufweist. Der Feuchtigkeitssensor 18 und/oder der Temperatursensor 19 kann/können in der Messvorrichtung 1 angeordnet sein, wie dies in Fig. 1 dargestellt ist. Es ist damit möglich, die Luftfeuchtigkeit und/oder die Temperatur während der Messung zu berücksichtigen, um das Ergebnis der Messung genauer bewerten zu können. Der Feuchtigkeitssensor 18 und/oder der Temperatursensor 19 kann/können beispielsweise in der Nähe des Aufnahmegefäßes 3 angeordnet sein.

Es ist aber auch möglich, dass Feuchtigkeitssensor 18 und/oder der Temperatursensor 19 oder eine zusätzlicher Feuchtigkeitssensor und/oder der Temperatursensor im Bereich der Vorrichtung zur Mischung der beiden Komponenten A und B miteinander oder in der Vorrichtung zur Verarbeitung des Gemisches aus den beiden Komponenten A und B angeordnet ist/sind und damit das Messergebnis besser auf die an diesen Orten vorherrschenden Bedingungen abstimmen zu können.

Das Messergebnis der Messung kann an eine externe Recheneinheit beispielsweise einen PC, übertragen werden. Bevorzugt erfolgt aber die Auswertung der Messung bereits in der Messvorrichtung 1. Dazu weist diese zumindest einen elektronischen Speicher auf, in dem die Daten aus der Messung und diverser Auswertungen gespeichert werden können.

Der zumindest eine elektronische Speicher kann Teil eines internen PC's sein, beispielsweise eines handelsüblichen Bedienpanels 20 (z.B. von der Fa. Beckhoff), wie dies in Fig. 3 dargestellt ist. Das Bedienpanel kann ein Multitouch-Einbau-Panel sein.

Für den Fall, dass die beiden Komponenten A und B an einem zum Ort der Messvorrichtung 1 verschiedenen Ort miteinander vermischt werden, kann nach einer weiteren Ausführungsvariante der Messvorrichtung 1 vorgesehen sein, dass der zumindest eine elektronische Speicher auch dazu verwendet wird, um eine Zeitspanne, die zwischen dem Mischen der Komponente A mit der Komponente B und dem Beginn der ersten Messung vergangen ist, zu berücksichtigen. Dazu kann die Messvorrichtung 1 ein Eingabeelement aufweisen, das mit dem elektronischen Speicher verbunden ist. Das Eingabeelement kann beispielsweise Teil des internen PC's, insbesondere des Bedienpanels 20, sein. Über dieses Eingabeelement wird die genannte Zeitspanne zwischen dem Mischen und dem Start der Messung eingegeben und im elektronischen Speicher hinterlegt.

Wie soeben beschrieben, kann die Mischung der beiden Komponenten A und B miteinander außerhalb der Messvorrichtung 1 erfolgen. Es kann aber auch vorgesehen sein, dass nach einer weiteren Ausführungsvariante der Messvorrichtung die Vermischung der beiden Komponenten A und B in der Messvorrichtung 1 selbst erfolgt. Dazu kann diese eine Mischvorrichtung 21 aufweisen, wie dies strichliert in Fig. 1 dargestellt ist. Die Mischvorrichtung 21 kann beispielsweise ein Rührer sein, beispielsweise mit Rührerwelle und Rührerblatt oder ein Magnetrührer. Die Mischvorrichtung 21 kann auch verstellbar in der Messvorrichtung 1 angeordnet sein, sodass sie nach dem Mischen aus dem Bereich des Aufnahmegefäßes 3 (automatisch) entfernt werden kann, um damit die Messung an sich nicht zu stören.

Gemäß einer weiteren Ausführungsvariante der Messvorrichtung 1 dazu kann vorgesehen sein, diese eine Zufuhrleitung 22 für die Komponente A und eine Zufuhrleitung 23 für die Komponente B zur Zufuhr der beiden Komponenten A und B in das Aufnahmegefäß 3 aufweist, wie dies ebenfalls in Fig. 1 strichliert dargestellt ist. Es kann damit der Automatisierungsgrad der Messung weiter erhöht werden. Die zuzuführenden Mengen können beispielsweise über den (internen) PC, insbesondere das Bedienpanel 20, festgelegt werden.

Vorzugsweise weist die Messvorrichtung 1 ein Gehäuse 24 auf. Dabei kann der Boden 9 Teil des Gehäuses 24 sein. Es ist aber auch möglich, dass das Gehäuse 24 größer ausgeführt ist, wie dies die Ausführungsvariante der Messvorrichtung 1 nach Fig. 3 zeigt. Dabei weist das Gehäuse einen Gehäusekäfig 25 auf, der mit entsprechenden Verkleidungselementen verkleidet ist. Der Boden 9 ist als Zwischenboden ausgeführt. Unterhalb des Bodens 9 kann eine Schublade 26 angeordnet sein, in der der interne PC, insbesondere das Bedienpanel 20, aufgenommen werden kann.

Darüber hinaus weist diese Messvorrichtung 1 wieder die Halterung 2 für das Aufnahmegefäß 3, das Halteelement 4 für das Messelement 5, die Halteeinrichtung 6, auf der das Halteelement 4 (linear) beweglich angeordnet ist, das Antriebselement 7 mit dem das Halteelement 4 (linear) verstellbar ist, und das Sensorelement 8 auf. Es sei dazu auf die voranstehenden Ausführungen zu diesen Bauteilen verwiesen.

Es sei angemerkt, dass zur besseren Übersichtlichkeit generell in den Figuren auf die Darstellung der Verkabelung der Messvorrichtung 1 verzichtet wurde. Diese ist selbstverständlich in der Messvorrichtung 1 vorhanden, wenngleich zumindest einzelne Bauteile der Messvorrichtung 1 auch drahtlose Datenübertragungsmodule aufweisen können.

Es kann ausreichend sein, nur das Ende der Topfzeit zu bestimmen. Mit der Messvorrichtung 1 kann aber nach einer Ausführungsvariante auch die Topfzeit an sich, also jene Zeitspanne, innerhalb der das härtende Gemisch aus den beiden Komponenten A und B verarbeitbar ist, ermittelt werden. Dazu wird die Topfzeit aus der Zeitspanne zwischen dem Mischen der Komponente A mit der Komponente B und dem detektierten Ende der Topfzeit, also dem Abreißen des Fadens 1 beim Herausziehen des Messelementes 5 aus dem Gemisch, errechnet. Die Berechnung kann in dem externen oder internen PC, insbesondere dem Bedienpanel 20 erfolgen. Für die Berechnung kann Zeitspanne, die zwischen der Mischung der Komponente A mit der Komponente B und dem Beginn der ersten Messung vergangen ist, in dem elektronischen Speicher hinterlegt und in die Berechnung der Topfzeit einbezogen werden, wie dies voranstehend ausgeführt wurde.

Es sei darauf hingewiesen, dass mit der ersten Messung jener Zeitpunkt gemeint ist, zu dem das Messelement 5 zum ersten Mal in das Gemisch aus den beiden Komponenten A und B abgesenkt wird.

Das Ergebnis der Messung der Topfzeit kann zusammen mit weiteren (überwachten) Parametern auf dem PC, insbesondere dem Bedienpanel 20, dargestellt bzw. mitprotokolliert werden.

Weitere Parameter sind die Art der gemischten Komponenten A und B, die Temperatur während der Messung, die Luftfeuchtigkeit während der Messung, die eingesetzte Menge an dem Gemisch, die Art und die Dauer der Durchmischung der Komponenten A und B miteinander, die Form des Aufnahmegefäßes 3, etc.

Es ist weiter möglich, vor der Messung eine Eichung durchzuführen. Dazu kann mit einer exakten Mischung der Komponenten A und B (hinsichtlich ihrer Mengen, beispielsweise im Verhältnis 1:10) die Topfzeit händisch bestimmt werden. Dieser Wert kann dann als Grundwert herangezogen werden, mit dem die Messergebnisse aus der Messung der Topfzeit mit der Messvorrichtung 1 verglichen werden können, um die Abweichung festzustellen bzw. gegebenenfalls eine der beiden Komponenten anders zu dosieren.

Um bei einer definierten Temperatur oder bei einer erhöhten Temperatur messen zu können, kann die Messvorrichtung 1 auch eine Heiz- und/oder Kühleinrichtung aufweisen, die gegebenenfalls einen Thermostat aufweist. Dabei kann das Aufnahmegefäß 3 auf dieser Heiz- und/oder Kühleinrichtung oder zumindest teilweise innerhalb dieser Heiz- und/oder Kühleinrichtung angeordnet sein.

Für die Messung kann das Sensorelement 8 in einer Höhe 27 über der Oberkante des Aufnahmegefäßes 3 angeordnet sein, die bevorzugt so ausgewählt ist, dass mit dem Messung auch noch ein Teil des Messelementes 5 miterfasst wird, wie dies in Fig. 4 ersichtlich ist. Die Höhe 27 bemisst sich dabei von der Oberkante des Aufnahmegefäßes 3 bis auf halbe Höhe des Messfeldes des Sensorelementes 3, das von diesem erfasst wird. Prinzipiell kann die Höhe 27, in der das Sensorelement 8 angeordnet wird, kann aber auch anders bemessen sein, wobei jedoch die Ausrichtung an die Unterkante des Messelementes 5 bevorzugt wird.

Die Messvorrichtung 1 kann weiter eine Statusanzeige aufweisen. Diese kann durch entsprechende Lampen bzw. Leuchtmittel gebildet sein, vorzugsweise mit unterschiedlichen Farben, um den Status der Messvorrichtung 1 schneller erfassen zu können. In der bevorzugten Ausführungsvariante der Messvorrichtung wird die Statusanzeige durch einen Schriftzug gebildet, beispielsweis den in Fig. 3 dargestellten Schriftzug. Hinter diesem befinden sich die Lampe bzw. Leuchtmittel oder befinden sich die Lampen oder die Leuchtmittel.

Die Statusanzeige kann beispielsweise wie folgt gestaltet werden:
- Weiß leuchtend: Messvorrichtung 1 eingeschaltet
- Blau leuchten: Während der Messung
- Blau blinken: während einer Messpause
- Rot blinken: Messung schlecht
- Grün blinken: Messung gut

Zusätzlich kann das Bedienpanel 20 als Anzeigeelement des Status der Messeinrichtung 1 dienen.

Die Messung der Topfzeit des Gemisches aus den beiden Komponenten A und B erfolgt vorzugsweise automatisch, d.h. insbesondere dass die Messzyklen (Absenken und Wiederanheben des Messelementes 5) automatisch so lange durchgeführt werden, bis der Faden 15 aus dem Gemisch reißt. Es kann aber auch ein manueller Betrieb vorgesehen werden.

Im Automatikmodus kann der Folgende Messablauf vorgesehen werden.

Das Messelement 5 bewegt sich nach unten, taucht mit der Spitze in das Gemisch ein und bleibt in dieser Position bis eine vordefinierbare Haltezeit für das Anheben des Messelementes 5 abgelaufen ist. Diese Haltezeit dient u.a. auch dazu, dass das an dem Messelement 5 anhaftende Harz bzw. die Reaktivmischung wieder mit der Masse im Aufnahmegefäß 3 in Kontakt kommt und damit eine weitere Reaktion der anhaftenden Menge ermöglicht wird, ohne die Aushärtung wesentlich zu stören. Aus diesem Grund ist auch die Halteposition des Messelementes 5 zwischen den Messungen die untere Endposition, in der das Messelement 5 in das Aufnahmegefäß 3 und damit in das Harz eintaucht, um das Gefüge, dass sich während des Härtens ausbildet, möglichst wenig zu stören.

Die Haltezeit kann beispielsweise ausgewählt werden aus einem Bereich von 20 Sekunden bis 200 Sekunden. Es kann z.B. eine Messung pro Minute ausgeführt werden (bis das Ende der Topfzeit erreicht ist).

Sobald diese die Haltezeit abgelaufen ist, wird das Messelement 5 um eine Länge X wieder angehoben und zieht den Faden 15 nach sich. Die Länge X kann dabei aus dem voranstehend genannten Bereich von 10 cm bis 50 cm ausgewählt werden. In der oberen Endposition der Messelementes 5 wird der Faden 15 von dem Sensorelement 8 erfasst, insbesondere von der genannten Kamera fotografiert und vermessen.

Anschließend fährt das Messelement 5 wieder nach unten in das Aufnahmegefäß. Das Messelement 5 kann aber auch bis zum nächsten Messzyklus in der oberen Endposition verbleiben, wenngleich dies nicht bevorzugt ist.

Dieser Ablauf (Messzyklus Anheben und Absenken des Messelementes 5 oder Absenken und Anheben des Messelementes 5, falls dieses in der oberen Endposition bis zum nächsten Messzyklus verbleibt) wird in vordefinierbaren Zeitspannen so lange wiederholt, bis entweder die eine voreingestellte maximale Messdauer erreicht ist oder der Faden 15 entsprechend abreißt. Diese Zeitspanne kann ausgewählt werden aus einem Bereich von 30 Sekunden bis 2 Minuten, insbesondere aus einem Bereich von 60 Sekunden bis 90 Sekunden.

Das Absenken des Messelementes 5 und/oder das Anheben des Messelementes 5 kann mit einer vordefinierbaren, insbesondere gleichförmigen, Geschwindigkeit erfolgen. Beispielsweise kann diese Geschwindigkeit für das Absenken und Eintauchen ausgewählt sein aus einem Bereich von 40 mm/s bis 100 mm/s und die Geschwindigkeit für das Rausziehen ausgewählt sein aus einem Bereich von 1200 mm/s bis 2000 mm/s.

Wie voranstehend bereits erwähnt, besteht die Möglichkeit, dass die Messung pausiert wird, beispielsweise um die Position des Aufnahmegefäßes 3 zu korrigieren.

Das Verfahren zur Messung der Topfzeit kann einerseits zur Überwachung der definierten Materialeigenschaften einer Charge/Lieferung der Komponenten A und B, und andererseits zur Überwachung der Prozessparameter der Fertigungs-/Dosieranlagen dienen. Es ist damit auch ein Verfahren zur Steuerung einer Vorrichtung zur Verarbeitung eines härtenden Gemisches aus einer Komponente A und einer Komponente B möglich, wonach mit der Messvorrichtung nach dem Verfahren die Topfzeit des Gemisches bestimmt wird und in Anhängigkeit der bestimmten Topfzeit des Gemisches die Zufuhr der Menge der Komponente A und der Menge der Komponente B zur Vorrichtung zur Verarbeitung des härtenden Gemisches aus der Komponente A und der Komponente B gleichbelassen oder automatisch verändert wird. Es kann also beispielsweise eine der beiden Komponenten A und B anders dosiert werden, falls die Topfzeit einen für die Verarbeitung des Gemisches nicht passenden Wert ergibt.

Im Voranstehenden wurde immer auf ein Gemisch aus den beiden Komponenten A und B (Zweikomponentensystem) Bezug genommen. Es ist aber auch möglich, dass das Gemisch mehr als zwei Komponenten enthält.

Darüber hinaus kann mit der Messvorrichtung 1 auch die Topfzeit eines sogenannten Einkomponentensystems, bei dem diese mit Luftsauerstoff oder Luftfeuchtigkeit härtet, gemessen werden. In diesem Fall entfällt die Herstellung des Gemisches aus den beiden Komponenten A und B.

In Fig. 4 sind der Vollständigkeit halber eine Reihe von Messelementen 5 dargestellt, wie sie bei der minütlichen Topfzeitmessung erfasst werden. Es sei daraufhingewiesen, dass das Messelement 5 während einer Messung bis zum Ende der Topfzeit nicht ausgetauscht wird, wie dies voranstehend ausgeführt wurde, auch wenn die Fig. 4 diesen Eindruck erweckt. Vielmehr tauch das Messelement 5 immer wieder in das Aufnahmegefäß 3 ein bis das Ende der Topfzeit erreicht ist.

Aus der Fig. 4 ist sehr gut erkennbar, wie sich der Faden 15 über die Dauer der Messung verändert. Die erste Messung zeigt dabei die rechte Darstellung des Messelementes 5, während das ganz linke Messelement 5 das Ende der Topfzeit darstellt. Mit fortschreitender Härtung nimmt die Länge des Fadens 15 ab. Diese Abnahme wird detektiert, indem das Sensorelement 8 (Fig. 1) auf Höhe der oberen Endlage des Messelementes im Bereich des anhaftenden Harzes positioniert wird. Damit erfasst das Sensorelement 8 die am Messelement 5 verbleibende Länge des Fadens 15 wenn dieser gerissen ist. Solange also im Messfeld des Sensorelementes 5 eine bestimmte, vordefinierbare Länge an dem Faden 15 vorhanden ist, erkennt die Messvorrichtung 1, dass die Topfzeit noch nicht zu Ende ist. Erst wenn beispielsweise nur mehr eine bestimmte, vordefinierbare Anzahl an Pixeln einen Faden 15 erfassen, und der Rest der Pixel eine andere Farbe oder eine andere Geometrie, etc., erfassen, kann die Messvorrichtung 1 auf das Ende der Topfzeit schließen, da damit nur mehr eine gewisse Länge des Fadens 15, wie er beispielsweise ganz links in Fig. 4 dargestellt ist, vorhanden ist.

Prinzipiell kann aber auch an einer anderen Stelle entlang der Weglänge während des Herausziehens des Messelementes 5 aus dem Aufnahmegefäß 3 gemessen werden. Bei entsprechender Länge des Sensorfeldes kann sogar die gesamte Wegstrecke beim Herausziehen des Messelementes 5 aus dem Aufnahmegefäß 3 erfasst werden.

Die verbleibende Länge des Fadens 15 am Messelement 5, wie sie beispielshaft am äußerst linken Messelement 5 in Fig. 4 dargestellt ist, kann vordefiniert werden, beispielsweise bestimmt anhand einer Eichmessung. Sie ist bei der dargestellten Ausführungsvariante das Maß für die Bestimmung des Endes der Topfzeit mit der Messvorrichtung 1.

Sollte das Sensorelement 8 an einer anderen Stelle positioniert sein, an der die Länge des Fadens 15 nicht mehr erfasst wird, dann kann sich das Ende der Topfzeit rein aus dem Nichtvorhandensein des Fadens 15 im Messfeld des Sensorelementes 8 nach dem Herausziehen des Messelementes 5 um eine bestimmte, vordefinierbare Länge aus dem Aufnahmegefäß 3 bestimmen.

Obwohl im Voranstehenden immer davon ausgegangen wurde, dass das Messelement 5 aus dem Aufnahmegefäß 3 herausgezogen wird, besteht auch die Möglichkeit, dass umgekehrt das Aufnahmegefäß 3 nach unten abgesenkt wird und das Messelement 5 stehend verbleibt.

Die Ausführungsbeispiele zeigen bzw. beschreiben mögliche Ausführungsvarianten, wobei an dieser Stelle bemerkt sei, dass auch diverse Kombinationen der einzelnen Ausführungsvarianten untereinander möglich sind.

Der Ordnung halber sei abschließend darauf hingewiesen, dass zum besseren Verständnis des Aufbaus der Messvorrichtung 1 diese bzw. deren Bestandtele nicht zwingenderweise maßstäblich dargestellt sind.

### Bezugszeichenaufstellung

- 1: Messvorrichtung
- 2: Halterung
- 3: Aufnahmegefäß
- 4: Halteelement
- 5: Messelement
- 6: Halteeinrichtung
- 7: Antriebselement
- 8: Sensorelement
- 9: Boden
- 10: Ausnehmung
- 11: Stirnfläche
- 12: Betätigungselement
- 13: Profilelement
- 14: Rückwand
- 15: Faden
- 16: Länge
- 17: Stützelement
- 17': Ausnehmung
- 18: Feuchtigkeitssensor
- 19: Temperatursensor
- 20: Bedienpanel
- 21: Mischvorrichtung
- 22: Zufuhrleitung
- 23: Zufuhrleitung
- 24: Gehäuse
- 25: Gehäusekäfig
- 26: Schublade
- 27: Höhe

## Patentansprüche

1. Verfahren zur Bestimmung des Endes der Topfzeit eines härtenden Harzes oder eines härtenden Gemisches aus einer Komponente A und einer Komponente B umfassend die Schritte:
- Gegebenenfalls vermischen der Komponente A mit der Komponente B zur Herstellung des Gemisches,
- Entnahme einer vorbestimmbaren Prüfmenge aus dem Harz oder dem Gemisch,
- Überführen der Prüfmenge in eine Messvorrichtung (1) zur Bestimmung der Topfzeit des Harzes oder des Gemisches,
- Eintauchen eines Messelementes (5) in vorbestimmbaren Zeitabständen in die Prüfmenge,
- anschließend daran Herausziehen des Messelementes (5) aus der Prüfmenge mit vorbestimmbarer Geschwindigkeit,
**dadurch gekennzeichnet, dass**
beim Herausziehen des Messelementes (5) ein Faden (15) aus dem Harz oder dem Gemisch gezogen wird, der mit einem Sensorelement (8) erfasst wird, wobei der Vorgang des Eintauchens und Herausziehen des Messelementes (5) solange wiederholt wird, bis der Faden (15) beim Herausziehen aus dem Harz oder dem Gemisch zur Gänze und mit einer vordefinierbaren verbleibenden Länge am Messelement (5) abreißt.

2. Verfahren nach Anspruch 1, **dadurch gekennzeichnet, dass** als Sensorelement (8) ein optisches Sensorelement verwendet wird.

3. Verfahren nach Anspruch 1 oder 2, **dadurch gekennzeichnet, dass** mit dem Sensorelement (8) die Geometrie des Fadens (15) im Messfeld des Sensorelementes (8) vermessen wird.

4. Verfahren nach einem der Ansprüche 1 bis 3, **dadurch gekennzeichnet, dass** die Topfzeit aus der Zeitspanne zwischen der Entnahme des Harzes oder dem Mischen der Komponente A mit der Komponente B und dem detektierten Ende der Topfzeit errechnet wird.

5. Verfahren nach Anspruch 4, **dadurch gekennzeichnet, dass** eine Zeitspanne, die zwischen der Entnahme des Harzes oder der Mischung der Komponente A mit der Komponente B und dem Beginn der ersten Messung vergangen ist, in einem elektronischen Speicher hinterlegt und in die Berechnung der Topfzeit einbezogen wird.

6. Verfahren nach einem der Ansprüche 1 bis 5, **dadurch gekennzeichnet, dass** mit zumindest einem Temperatursensor (19) die Umgebungstemperatur der Messvorrichtung (1) und/oder die Temperatur beim Mischen der Komponente A mit der Komponente B erfasst wird oder beim Verarbeiten des Harzes oder des Gemisches.

7. Verfahren nach einem der Ansprüche 1 bis 6, **dadurch gekennzeichnet, dass** mit zumindest einem Feuchtigkeitssensor (18) die Luftfeuchtigkeit der Umgebung der Messvorrichtung (1) und/oder der Luftfeuchtigkeit beim Mischen der Komponente A mit der Komponente B oder beim Verarbeiten des Harzes oder des Gemisches erfasst wird.

8. Messvorrichtung (1) zur Durchführung des Verfahrens nach einem der vorhergehenden Ansprüche zur Bestimmung des Endes der Topfzeit eines härtenden Harzes oder eines härtenden Gemisches aus einer Komponente A und einer Komponente B umfassend:
- eine Halterung (2) für ein Aufnahmegefäß (3), in das eine Prüfmenge des Harzes oder des Gemisches einfüllbar ist,
- ein Halteelement (4) für ein Messelement (5),
- eine Halteeinrichtung (6), auf der das Halteelement (4) beweglich, insbesondere linear beweglich, angeordnet ist,
- ein Antriebselement (7) mit dem das Halteelement (4) verstellbar, insbesondere linear verstellbar, ist,
- ein Sensorelement (8),
**dadurch gekennzeichnet, dass** das Halteelement (4) um eine Länge von mindestens 10 cm verstellbar, insbesondere linear verstellbar, ist.

9. Messvorrichtung (1) nach Anspruch 8, **dadurch gekennzeichnet, dass** das Sensorelement (8) ein optisches Sensorelement ist.

10. Messvorrichtung (1) nach Anspruch 8 oder 9, **dadurch gekennzeichnet, dass** das optische Sensorelement eine Kamera mit einem CCD-Sensor ist.

11. Messvorrichtung (1) nach einem der Ansprüche 8 bis 10, **dadurch gekennzeichnet, dass** diese zusätzlich zumindest einen Feuchtigkeitssensor (18) und/oder zumindest einen Temperatursensor (19) aufweist.

12. Messvorrichtung (1) nach einem der Ansprüche 8 bis 11, **dadurch gekennzeichnet, dass** diese einen elektronischen Speicher aufweist.

13. Messvorrichtung (1) nach Anspruch 12, **dadurch gekennzeichnet, dass** der elektronische Speicher mit einem Eingabeelement verbunden ist, über das eine Zeitspanne eingegeben werden kann, die zwischen der Entnahme des Harzes oder zwischen dem Mischen der Komponente A mit der Komponente B und dem Beginn der ersten Messung vergangen ist.

14. Messvorrichtung (1) nach einem der Ansprüche 8 bis 13, **dadurch gekennzeichnet, dass** diese eine Mischvorrichtung (21) für die Vermischung der Komponente A mit der Komponente B aufweist.

15. Messvorrichtung (1) nach einem der Ansprüche 8 bis 14, **dadurch gekennzeichnet, dass** diese eine Zufuhrleitung (22) für die Komponente A und eine Zufuhrleitung (23) für die Komponente B zur Zufuhr der beiden Komponenten A und B in ein Aufnahmegefäß (3) aufweist.

16. Verfahren zur Steuerung einer Vorrichtung zur Verarbeitung eines härtenden Gemisches aus einer Komponente A und einer Komponente B, wonach mit einer Messvorrichtung (1) nach einem der Ansprüche 8 bis 15 nach einem Verfahren entsprechend einem der Ansprüche 1 bis 7 die Topfzeit des Gemisches bestimmt wird und in Anhängigkeit der bestimmten Topfzeit des Gemisches die Zufuhr der Menge der Komponente A und der Menge der Komponente B zur Vorrichtung zur Verarbeitung des härtenden Gemisches aus der Komponente A und der Komponente B gleichbelassen oder verändert wird.
